# EUROPÄISCHE PATENTSCHRIFT

(11) **EP 1 063 959 B1**
(45) Veröffentlichungstag und Bekanntmachung des Hinweises auf die Patenterteilung: **06.11.2002**
(21) Anmeldenummer: 99908953.5
(22) Anmeldetag: 10.03.1999
(51) Int. Cl.: A61K 7/42

(54) **SONNENSCHUTZFORMULIERUNGEN MIT WIRKUNG GEGEN HERPES SIMPLEX VIREN**
SUN-PROTECTION FORMULATIONS ACTIVE AGAINST HERPES SIMPLEX VIRUSES
FORMULATIONS DE PROTECTION SOLAIRE A ACTION CONTRE LES VIRUS DE L'HERPES SIMPLEX

(30) Priorität: 18.03.1998 DE 19811692
(43) Veröffentlichungstag der Anmeldung: 03.01.2001
(73) Patentinhaber: MERCK PATENT GmbH, 64293 Darmstadt (DE)
(72) Erfinder: DRILLER, Hansjürgen, D-64853 Otzberg (DE); HITZEL, Sabine, D-64409 Messel (DE)
(86) Internationale Anmeldenummer: EP9901535
(87) Internationale Veröffentlichungsnummer: WO99047109

(56) Entgegenhaltungen:
- EP-A- 0 046 451
- EP-A- 0 898 955
- DE-A- 4 229 713

## Beschreibung

Die vorliegende Erfindung betrifft die Verwendung Sonnenschutzformulierungen in fester oder flüssiger Form, enthaltend anorganische Lichtschutzfilter mit prophylaktischer Wirkung gegen herpititformen Erkrankungen der Haut.

Bekanntlich reagiert die Haut empfindlich auf Sonnenstrahlen, welche einen gewöhnlichen Sonnenbrand oder ein Erythem, aber auch mehr oder weniger ausgeprägte Verbrennungen hervorrufen können.

Sonnenstrahlen haben aber auch andere negative Wirkungen: Sie bewirken, daß die Haut ihre Elastizität verliert und sich Falten bilden, somit führen sie zu einer frühzeitigen Alterung. Manchmal kann man auch Dermatosen beobachten. Im extremen Fall kann es bei manchen Menschen zum Auftreten von Hautkrebs kommen.

Weiterhin ist bekannt, daß bei Menschen, welche mit Herpes Viren infiziert sind und unter Streß an Herpes leiden, nach intensiver Sonnenbestrahlung in vielen Fällen die Herpesviren aktiviert werden und an herpititformen Erkrankungen der Haut, insbesondere im Mund- und Lippenbereich Bereich, leiden.

Der gefährlichste Teil der Sonnenstrahlen wird von den ultravioletten Strahlen mit einer Wellenlänge von weniger als 400 nm gebildet. Die Mehrzahl der unerwünschten Wirkungen des Sonnenlichts wie z. B. Sonnenbrand, lichtinduzierte Zellschäden und Hautkrebs werden durch UV-B-Strahlung (280 - 320 nm) hervorgerufen

UV-B-Strahlung kann über drei verschiedene Reaktionswege Hautrötung und Schädigung der Zellkerne hervorrufen:
- Sonnenbrand durch Freisetzung von biologischen "Botenstoffen",
- Hautkrebs durch direkte Schädigung der DNS des Zellkerns,
- Hautkrebs durch die Freisetzung von freien Radikalen.

Ein Sonnenbrand ist an sich ausschließlich die Folge einer Überdosis von UV-B-Strahlung. Diese Strahlung setzt biochemische Botenstoffe wie z. B. Histamin frei, die ihrerseits die bekannten Wirkungen wie Jucken, Schmerz- und Hitzegefühl und das "Brennen" der Haut hervorrufen. Die Botenstoffe diffundieren auch in die Blutgefäße, verursachen dort eine Erweiterung, und rufen so ein Ödem hervor. Zusätzlich bewirken sie eine Proliferation der Basalzellen.

Sonnenbestrahlung, auch solche, die noch nicht zu einem Sonnenbrand führt, bedeutet also für die Hautzellen Streß, durch die die Abwehrmechanismen gegen Infektionen, bzw die unerwünschte Wirkung von Bakterien oder Viren geschwächt werden. Eine latente Herpesinfektion kann so aktiviert werden und sich in der Bildung von schmerzhaften Bläschen zeigen.

Die Wirkungen einer Überdosierung von UV-B-Bestrahlung werden frühestens etwa 4 Stunden nach der Bestrahlung beobachtet, zu spät also für jegliche Gegenmaßnahmen. Ein Sonnenbrand ist deshalb kein Warnsignal, sondern das Zeichen einer bereits vorliegenden Hautschädigung.

UV-A-Strahlung mit Wellenlängen größer als 340 nm ist bei gesunder Haut hauptsächlich für den Prozeß der Hautalterung verantwortlich. Daneben wird aber auch für die UV-A-Strahlung eine karzinogene Wirkung diskutiert.

Bekannt ist auch, daß durch das Vorhandensein der Ozonschicht der Erdatmosphäre, die einen Teil der Sonnenstrahlung absorbiert, die untere Grenze der ultravioletten Strahlen, welche die Erdoberfläche erreichen, bei ca. 280 nm liegt. Chronische Zellschädigungen wie Hautalterung und Hautkrebs werden durch direkte Schädigung des Zellkerns hervorgerufen, der eine spezifische Empfindlichkeit für UV-Strahlung mit Wellenlängen unterhalb 320 nm aufweist. Erst bei Wellenlängen oberhalb 340 nm absorbiert er keine Strahlung mehr. Schädigungen des Zellkerns beruhen auf Schädigungen der DNS. Dieses führt zum Verlust der Funktionsfähigkeit der DNS, gleich in welcher Weise die Schädigung erfolgt.

Der Wellenlängenbereich des Sonnenlichts erstreckt sich jedoch nicht nur über den Bereich der UVA- und UVB-Strahlung von 280 bis 400 nm. Der durch das Auge wahrnehmbare Bereich reicht bis zu 800 nm und ist begrenzt durch den Übergang in die langwellige Infrarotstrahlung, welche als Wärmestrahlung wahrgenommen wird. Nach unten geht die Strahlung über in den kurzwelligen UV-Bereich, auch UVC-Strahlung genannt. Dieses ist der Wellenlängenbereich von 100 bis 280 nm.

Patent Dokument DE-A-42 29 713 offenbart die Verwendung einer Sonnenschutzformulierung, die organische Lichtschutzfilter enthält, die im UV-Bereich absorbieren, zur Herstellung eines Arzneimittels zur Prophylaxe gegen Herpes labialis.

Sonnenlicht im Wellenlängenbereich von 400 bis 800 nm (VlS-Bereich) kann in tiefere Hautschichten eindringen und dort in schädigender Weise wirksam werden, so daß gerade auch durch diesen Einfluß des Lichts Hautalterungsprozesse beschleunigt werden können. Dieses gilt auch für IR-Strahlung. Nicht nur UV-A- und UV-B-Strahlung wirken also schädigend auf die Hautzellen ein und rufen neben direkt sichtbaren kurzzeitigen Schäden, wie Sonnenbrand und Herpes, langfristige Hautveränderungen und -schäden hervor, auch Strahlung im Wellenlängenbereich des sichtbaren Lichts (VIS-Bereich) und im Bereich der Infrarotstrahlung (IR-Bereich) trägten zur Schädigung der Haut bei und schwächten ihre Abwehrmechanismen.

Es erscheint somit wünschenswert, Sonnenschutzformulierungen zur Verfügung zu stellen, die einen möglichst umfassenden Schutz gegen den hautschädigenden Einfluß durch Sonnenbestrahlung gewährleisten. Insbesondere ist es aber auch wünschenswert, solche Sonnenschutzformulierungen zur Verfügung zu stellen, die eine prophylaktische Wirkung gegen herpititforme Erkrankungen der Haut aufweisen.

Gegenstand der Erfindung sind somit Sonnenschutzformulierungen, enthaltend anorganische Lichtschutzfilter mit prophylaktischer Wirkung gegen herpititformen Erkrankungen der Haut, insbesondere der Haut im Mund- und Lippenbereich.

Erfindungsgemäße Sonnenschutzformulierungen weisen eine prophylaktischer Wirkung gegen herpititformen Erkrankungen der Haut, hervorgerufen durch Viren der Gruppe Herpes simplex und Herpes labialis auf.

Gegenstand der Erfindung ist die Verwendung von Sonnenschutzformulierungen, welche anorganische Lichtschutzfilter enthalten, zur Prophylaxe gegen herpititforme Erkrankungen der Haut, insbesondere gegen solche Erkrankungen der Haut im Mund- und Lippenbereich, welche hervorgerufen werden durch Viren der Gruppe Herpes simplex und Herpes labialis.

Die Verwendung von anorganischen Lichtschutzfiltern enthaltenden und im UVA-, UVB-, IR- und sichtbaren Bereich des Lichtspektrums absorbierenden Sonnenschutzformulierungen zur Prophylaxe gegen herpititformen Erkrankungen der Haut hervorgerufen durch Viren der Gruppe Herpes simplex und Herpes labialis ist Gegenstand der Erfindung.

Die heute üblichen Sonnenschutzfilter in der Kosmetik werden in UVA- bzw. UVB-Filter unterteilt. Für beide UV-Bereiche gibt es viele aus der Fachliteratur bekannte und bewährte Substanzen, anorganische Lichtschutzfilter aus der Gruppe Titandioxid und Zinkoxid aufgeführt.

Im Wellenlängenbereich von 400 bis 800 nm wirkende Lichtschutzfilter (VIS-Filter) können in kosmetischen Formulierungen lösliche, unlösliche oder Gemische von unlöslichen Substanzen sein. Es kann sich hierbei um im sichtbaren Wellenlängenbereich reflektierende und oder absorbierende (VIS-reflektierende) Pigmente handeln. Solche Pigmente können insbesondere goldene, rote, orange-, kupferoder körperfarbene Interferenzpigmente sein, welche der natürlichen Hautfarbe sehr nahe kommen.

Bei den Interferenzpigmenten handelt es sich bevorzugt um plättchenförmigen oder vermahlenen Glimmer mit einem Durchmesser von bis zu 15 µm, welcher beschichtet ist mit SnO₂ und/oder TiO₂. Es sind aber auch Interferenzpigmente geeignet, deren Trägermaterial nicht aus Glimmer besteht. Die Beschichtungen können unterschiediich dotiert sein, wie z. B. durch Eisen oder Cer.

In einer besonderen Ausführungsform solcher als VIS-Filter geeigneter Pigmente handelt es sich um Glimmer mit einer dünnen Beschichtung, bestehend aus bis zu einem Gew.-% SnO₂, und einer Beschichtung, bestehend aus 50 - 70 Gew.-%, bevorzugt 54 - 60 Gew.-%, TiO₂ mit einer Rutil-Struktur.

Es kann sich auch um Glimmer mit einer dünnen Beschichtung, bestehend aus bis zu einem Gew.-% SnO₂, und einer Beschichtung, bestehend aus 50 - 70 Gew.-%, bevorzugt 54 - 60 Gew.-%, TiO₂ mit einer Anatas-Struktur handeln oder um Glimmer mit einer Beschichtung, bestehend aus 50 - 70 Gew.-%, bevorzugt 54 - 60 Gew.-%, TiO₂ mit einer Anatas-Struktur.

Geeignete Substanzen, die in kosmetischen Formulierungen eingesetzt werden können und als VIS-Filter wirksam sind, sind Perlglanzpigmente, bestehend aus Glimmer oder anderen Trägermaterialien, welche mit Titandioxiden oder Eisenoxiden belegt sind, insbesondere sind dieses
- Silberpigmente (Glimmer + TiO₂) mit Partikelgrößen < 200µm insbesondere < 15 µm, wie z. B. das im Handel erhältliche Timiron MP 1005® oder MP 1001® oder auch gröbere Fraktionen
- Interferenzpigmente (Glimmer + TiO₂) mit Partikelgrößen <200µm insbesondere < 5 - 25 µm mit goldener, roter, orange-, kupfer- oder körperfarbener Interferenz, wie z. B. Timiron Silk Red® oder Silk Gold® oder Super Red® und Super Gold® oder Super Copper® oder gröbere Fraktionen oder andere Interferenzfarben und deren Mischungen
- Goldpigmente (Glimmer mit TiO₂ und Eisenoxide) mit Partikelgrößen < 200µm insbesondere < 5 - 25 µm oder < 15 µm; ein solches Goldpigment ist z.B. Timiron MP 20® , aber auch gröbere Goldpigmentfraktionen sind geeignet.
- Farbpigmente (Glimmer mit TiO₂ und Eisenoxide) mit Partikelgrößen < 200µm insbesondere < 5 - 25 µm oder < 15 µm. Entsprechende Farbpigmente sind z. B. Dichrona® oder Microna® Matte

Geeignet sind auch VlS-absorbierende oder -reflektierende Füllstoffe, wie z. B Glimmer belegt mit TiO₂ und/oder BaSO₄. Hierzu zählen auch z. B. Biron® (BiOCI), Low Luster® oder Extender W® , sofern sie nicht 100 %ig transparent sind.

Als anorganische UV-Filter sind mikrofeine ZnO- und TiO₂-Partikel geeignet, die gegebenenfalls auch im VIS-Bereich reflektieren oder absorbieren. Im Handel erhältlich sind diese unter den Namen Hombitec® oder Sachtotec® , Kemira M160® Tioveil AQ® und eingeschränkt Eusolex T-2000® , eingeschränkt, da es eine sehr hohe Transparenz aufweist.

Diese Substanzen bieten den Vorteil, keine toxischen oder allergischen Reaktionen gegenüber der Haut zu zeigen. Sie besitzen einen hohen Lichtschutzfaktor und damit eine langanhaltende Schutzwirkung. Neben ihrer Schutzwirkung im VIS-Bereich können sie auch eine Schutzwirkung im UV- oder IR-Bereich besitzen.

Als VIS-Filter können auch in der Kosmetik zugelassene Farbstoffe wirksam sein, beispielsweise ausgewählt aus der "Blauen Liste" (Liste der in der Kosmetik zugelassenen Farbstoffe) ["Blaue Liste" Editio Cantor Verlag, Hrsg. H. P. Fiedler,(1993)], die einzeln aber auch im Gemisch eingesetzt werden können. Diese Farbstoffe können als ungelöste Pigmente eingesetzt werden. Insbesondere kommen hier die roten, gelben und blauen Farbstoffe in Frage, die einzeln oder im Gemisch mit den übrigen Zusätzen zu Formulierungen führen, welche beim Auftragen auf die Haut eine natürliche Färbung zeigen. Daher sind auch Farbstoffe dieser Liste mit anderen als den genannten Farben einsetzbar, wie z. B. orange oder gold. Bevorzugt eingesetzt werden als rote Farbstoffe die der Namen D&C Red, vorzugsweise mit den Nummern No. 10, C.l. 15630, No. 7, C.l. 15850 und No. 21, C.l. 45380, Acid Red, vorzugsweise Acid Red 1, C.l. 18050, Allura Red, trans-alpha-,beta- oder gamma-Carotin, Pigment Red. Als gelbe Farbstoffe sind dieses jene mit den Namen Acid Yellow, vorzugsweise Acid Yellow 1, C.l. 10316, Tartrazine, C.l. 19140, Rutin, D & C Yellow No. 7, C. l. 45350, Disperse Yellow, Food Yellow, Natural Yellow, Pigment Yellow, Solvent Yellow. Entsprechende blaue Farbstoffe sind Acid Blue, vorzugsweise Acid Blue 9, C.l. 42090, Acid Blue 80, C.l. 61585, D & C Blue No. 6, C.l. 73000, C-Blau 21, Direct Blue 86.

Außer den in der genannten Liste aufgezählten Farbstoffen kommen auch weitere VlS-absorbierende Substanzen in Frage, wie z. B. Flavonoide oder natürliches oder künstliches Melanin.

Als anorganische UV-Filter können weiterhin dem Fachmann allgemein bekannte UV-Filter wie zum Beispiel solche aus der Gruppe Titandioxid und Zinkoxid eingesetzt werden.

Geeignete VIS-Filter können in Konzentrationen von 0,5 bis etwa 20 Gew.-% in kosmetische Formulierungen eingearbeitet werden. Es ist auf diese Weise möglich Formulierungen herzustellen, in denen bis zu 100 % der eingesetzten Lichtschutzfilter VIS-Filter sind. Es handelt sich hierbei um Substanzen, die mit Wasser und Ölen in einfacher Weise gelöst, dispergiert oder emulgiert werden können.

Die erfindungsgemäßen Formulierungen, die in einem sehr breiten Wellenlängenbereich einen wirksamen Schutz gewährleisten, können somit zur vorbeugenden Behandlung von Entzündungen und Allergien der Haut, zur Verhütung bestimmter Krebsarten gegebenenfalls auch zur Insektenabwehr und insbesondere zur Vorbeugung gegen herpititforme Erkrankungen der Haut verwendet werden. Bereits die Verwendung von Sonnenschutzformulierungen mit einem intensiven Schutz gegen UV-A- und UV-B-Strahlung zeigt hier eine gute Wirkung gegen die Aktivierung von Herpesviren, welche allgemein als Herpes simplex bekannt sind. Für den Mund- und Lippenbereich haben sich in durchgeführten Versuchen Formulierungen als hervorragend erwiesen, welche auch einen guten VIS-Schutz gewährleisten und in dem sichtbaren Bereich absorbierende Lichtschutzfilter enthalten. Letzteres sind anorganische Filter. Geeignet sind z. B. wie oben beschrieben Lichtschutzfilter auf der Basis von Titandioxid und Zinkoxid. Bei dem Titandioxid kann es sich um mikronisiertes Titandioxid handeln, welches im Handel unter dem Namen Eusolex T-2000 erhältlich ist.

Durch geeignete Wahl der Lichtschutzfilter, bzw. der Kombination an Lichtschutzfiltern und gegebenenfalls weiterer Zusätze verteilen sich die wirksamen Komponenten der erfindungsgemäßen Sonnenschutzformulierungen gleichmäßig in den herkömmlichen kosmetischen Trägern und können insbesondere in Fettträgern einen kontinuierlichen Film bilden; sie können auf diese Weise auf die Haut aufgetragen werden, um einen wirksamen Schutzfilm zu bilden.

Die erfindungsgemäße Entdeckung ist nicht aus dem Stand der Technik durch einfache Schlußfolgerung ableitbar, da üblicherweise Sonnenschutzformulierungen so zusammengesetzt sind, daß sie im UV-B- und in der letzten Zeit auch im UV-A-Bereich Strahlung absorbieren, nicht dagegen im sichtbaren Wellenlängenbereich von 400 bis 800 nm (VIS-Bereich) und im IR-Bereich, da letztere Strahlungsarten bisher als harmlos für die Haut erachtet wurden. Wie aber gefunden wurde, besitzt gerade auch eine Bestrahlung in diesem Wellenlängenbereich des Lichts eine schädigende Wirkung. Der eigentliche Schädigungsmechanismus ist zwar nicht eindeutig geklärt, sicher ist jedoch, daß diese Strahlung in tiefere damit aktivere Hautschichten eindringen und dort in schädigender Weise wirksam werden, so daß gerade auch durch diesen Einfluß des Lichts Hautalterungsprozesse beschleunigt werden kann. Dieses bedeutet, gleichgültig, ob die Haut nun durch Licht im UV-, sichtbaren oder lR-Wellenlängenbereich bestrahlt wird, es treten immer schädigende Wirkungen für die Zellen der Haut auf. Dadurch sind auf der einen Seite in den Zellen Regenerations- und Schutzreaktionen erforderlich. Gleichzeitig werden durch die schädigende Wirkung Abwehrreaktionen der Haut gegen Bakterien und Viren geschwächt. Bei bereits erfolgter Infektion mit Herpesviren bzw. bei Personen mit latenter Herpesinfektion kann dieses zu einer Aktivierung der Herpesviren führen und herpititforme Erkrankungen der Haut hervorrufen. Insbesondere betreffen diese Erkrankungen die Haut im Mund- und Lippenbereich und werden von Viren der Gruppe Herpes simplex, wobei die im Mund und Lippenbereich aktive Form zum HSV-1 Typ zählt und als Herpes labialis bezeichnet wird, hervorgerufen.

Es wurde nun gefunden, daß diese unerwünschten Erkrankungen der Haut vermieden werden können, wenn auf die Haut, insbesondere vor intensiver Sonnenbestrahlung, ein Sonnenschutzmittel aufgetragen wird, welches in einem sehr weiten Wellenlängenbereich Strahlung absorbiert und so zu einer möglichst geringen Schädigung der Hautzellen führt.

In Abhängigkeit vom Wellenlängenbereich, in welchem sie absorbieren, können die Lichtschutzfilter in den erfindungsgemäßen Sonnenschutzformulierungen, einzeln oder in Kombination mit einem oder mehreren Lichtschutzfiltern anderer Substanzklassen vorliegen, die jeweils in einer Menge von 0.01 bis 40 Gew.-%, vorzugsweise von 0.5 bis 20 Gew.-% bezogen auf das Gesamtgewicht der kosmetischen Zubereitung, enthalten sein können. Eine weitere ganz besonders bevorzugte Ausführungsform enthält 3 bis 10 Gew.-%. Wie beschrieben, können die Lichtschutzfilter bis zu 100 % durch die VIS-Filter ersetzt sein, sofern sie einen wirksamen Schutz in einem sehr weiten Wellenlängenbereich bieten. Insbesondere können, wie oben gesagt, auch Kombinationen von verschiedenen Lichtschutzfiltern eingesetzt werden, die sowohl anorganisch als auch organisch sein können.

Durch gezielte Auswahl der Einzelkomponenten weisen die erfindungsgemäßen Sonnenschutzformulierungen eine hohe chemische Stabilität, d.h. keine Hydrolysierbarkeit, keine Oxidierbarkeit, hohe Thermostabilität und hohe Schweißfestigkeit auf.

Die erfindungsgemäßen Sonnenschutzformulierungen zum Schutz menschlicher Epidermis gegen den schädlichen Einfluß von Sonnenbestrahlung können, je nach Verwendung, in verschiedenen, für diesen Typ üblicherweise verwendeten Formen vorliegen. So können sie insbesondere in Form öliger, ölig-wäßriger, wäßrig-alkoholischer oder ölig-alkoholischer Lotionen, Emulsionen, wie als Creme oder als Milch (W/O oder O/W), in Form ölig-alkoholischer, ölig-wäßriger oder wäßrig-alkoholischer Gele, Dispersionen oder als feste Stifte oder Puder vorliegen oder als Spray oder Aerosol konfektioniert sein.

Die erfindungsgemäßen Formulierungen können weitere kosmetische Adjuvanzien enthalten, welche in dieser Art von Zubereitungen üblicherweise verwendet werden, wie z.B. Verdickungsmittel, weichmachende Mittel, Befeuchtungsmittel, grenzflächenaktive Mittel, Konservierungsmittel, Mittel gegen Schaumbildung, Parfüms, Wachse, Lanolin, Treibmittel, Farbstoffe und/oder Pigmente, welche das Mittel selbst oder die Haut färben, und andere in der Kosmetik gewöhnlich verwendete Ingredienzien.

Man kann als Solubilisierungsmittel ein Öl, Wachs oder sonstigen Fettkörper, einen niedrigen Monoalkohol oder ein niedriges Polyol oder Mischungen davon verwenden. Zu den besonders bevorzugten Monoalkoholen oder Polyolen zählen Ethanol, i-Propanol, Propylen-glycol, Glycerin und Sorbit.

Eine bevorzugte Ausführungsform der Erfindung ist eine Emulsion, welche als Schutzcreme oder -milch vorliegt und außer einem oder mehreren Lichtschutzfaktoren, mindestens einen VIS-Filter, Fettalkohole, Fettsäureester, insbesondere Triglyceride von Fettsäuren, Fettsäuren, Alkylpolyglycoside, Lanolin, natürliche oder synthetische Öle oder Wachse und Emulgatoren in Anwesenheit von Wasser umfaßt.

Weitere bevorzugte Ausführungsformen stellen ölige Lotionen auf Basis von natürlichen oder synthetischen Ölen und Wachsen, Lanolin, Fettsäureestern, insbesondere Triglyceriden von Fettsäuren, oder ölig-alkoholische Lotionen auf Basis eines Niedrigalkohols, wie Ethanol, oder eines Glycols, wie Propylenglykol, und/oder eines Polyols, wie Glycerin, und Ölen, Wachsen und Fettsäureestern, wie Triglyceriden von Fettsäuren, dar.

Die erfindungsgemäße Sonnenschutzformulierung kann auch als alkoholisches Gel vorliegen, welches einen oder mehrere Niedrigalkohole oder -polyole, wie Ethanol, Propylenglycol oder Glycerin, und ein Verdickungsmittel, wie Kieselerde umfaßt. Die ölig-alkoholischen Gele enthalten außerdem natürliches oder synthetisches Öl oder Wachs.

In einer Dispersion kann als Dispersionsmittel ein Öl, Wachs oder sonstiger Fettkörper, ein niedriger Monoalkohol oder ein niedriges Polyol oder Mischungen davon verwendet werden. Zu den besonders bevorzugten Monoalkoholen oder Polyolen zählen Ethanol, i-Propanol, Propylenglycol, Glycerin und Sorbit.

Eine besondere Ausführungsform der vorliegenden Erfindung besteht in festen Stiften, welche aus natürlichen oder synthetischen Wachsen und Ölen, Fettalkoholen, Fettsäuren, Fettsäureestern, Lanolin und anderen Fettkörpern bestehen und neben pflegenden Zusätzen insbesondere anorganische Lichtschutzfilter enthalten, die einen möglichst breiten Wellenlängenbereich des Sonnenlichts absorbieren.

Ist eine Sonnenschutzformulierung als Aerosol konfektioniert, verwendet man in der Regel die üblichen Treibmittel, wie Alkane, Fluoralkane und Chlorfluoralkane.

Ist die erfindungsgemäße Sonnenschutzformulierung als Spray konfektioniert, verwendet man in der Regel wäßrig-alkoholische Lösungen.

Gegebenenfalls können den Formulierungen eine oder mehrere chemische Substanzen mit selbstbräunenden Eigenschaften hinzugefügt sein. Als chemische Substanzen mit selbstbräunenden Eigenschaften können alle dem Fachmann bekannten natürlichen und synthetischen Substanzen, welche zur Herstellung von kosmetischen Formulierungen geeignet sind, eingesetzt werden. Solche können sowohl pflanzliche Extrakte als auch synthetische Selbstbräuner, wie z. B. Dihydroxyaceton oder α-Ketole sein.

Die erfindungsgemäßen Sonnenschutzformulierungen können mit Hilfe von Techniken hergestellt werden, die dem Fachmann wohl bekannt sind.

Auch ohne weitere Ausführungen wird davon ausgegangen, daß ein Fachmann die obige Beschreibung in weitesten Umfang nutzen kann. Die bevorzugten Ausführungsformen sind deswegen lediglich als beschreibende, keineswegs als in irgendeine Weise limitierende Offenbarung aufzufassen.

Dieses gilt auch für das nachfolgende Beispiel, welches lediglich zum besseren Verständnis der vorliegenden Erfindung beitragen soll und daher nicht geeignet ist die Gültigkeit der vorliegenden Erfindung auf dieses Beispiel zu begrenzen.

### Beispiele

### Sonnenschutzcreme (W/O)

### Sonnenschutzfactor 20

| | | **%** |
|---|---|---|
| **A Eusolex T-2000 (Art.-Nr. 1.05373)** | **mikron. Titandioxid** | **(1) 3,00** |
| **Eusolex OCR (Art.-Nr. 1.05377)** | **Octocrylene** | **(1) 4,00** |
| Dehymuls E | Dicocoyl Pentyerythrityl Citrate and Sorbitan Sesquioleate (and) Cera Alba (and) Aluminium Stearate | (2) 6,00 |
| | | |
| Arlacel 989 | PEG-7 Hydrogenated Castor Oil | (3) 1,00 |
| | | |
| Bienenwachs (Art. Nr. 1.11544) | Cera Alba | (1) 2,00 |
| Zinkstearat (Art. Nr. 1.08865) | Zinc Stearate | (1) 2,00 |
| Cetiol J 600 | Oleyl Erucate | (2) 6,00 |
| Cetiol V | Decyl Oleate | (2) 6,00 |
| Cetiol OE | Dicaprylyl Ether | (2) 5,00 |
| Dow Corning 200 (100cs) | Dimethicone | (4) 1,00 |
| DL-α-Tocopherolacetat | Tocopheryl Acetate | (1) 1,00 |

| **B Eusolex 232 (Art.-Nr. 1.05372)** | **Phenylbenzimidazole Sulfonic Acid** | **(1) 2,00** |
|---|---|---|
| Tris(hydroxymethyl)-aminomethan (Art.-Nr. 1.08386) | Tromethamine | (1) 0,88 |
| | | |
| Glycerin (etwa 87 %) (Art.-Nr. 1.04091) | Glycerin | (1) 5,00 |
| | | |
| Magnesiumsulfat Heptahydrat (Art.-Nr. 1.05882) | Magnesium Sulfate | (1) 1,00 |
| Allantoin (Art.-Nr. 1.01015) | Allantoin | (1) 0,20 |
| Konservierungsmittel | | q.s. |
| Wasser, demineralisiert | | ad 100,00 |

### Herstellung:

Zur Neutralisierung von Eusolex 232 wird das Tris(hydroxymethyl)-aminomethan im Wasser der Phase B gelöst und Eusolex 232 unter Rühren zugegeben. Nach vollständiger Lösung werden die restlichen Rohstoffe der Phase B zugegeben und auf 80 °C erhitzt. Phase A auf 75 °C erhitzen. Phase B langsam in Phase A einrühren und unter Rühren abkühlen.

### Bemerkungen:

Viskosität 83.000 mPas (Brookfield RVT, Sp. C, 10 Upm) bei 25 °C
Muster enthalten als Konservierungsmittel
0,05 % Propyl-4-hydroxybenzoat (Art. Nr. 1.07427)
0,15 % Methyl-4-hydroxybenzoat (Art. Nr. 1.06757)

### Bezugsquellen:

(1) Merck KGaA, Darmstadt
(2) Henkel KGaA, Düsseldorf
(3) ICI, Essen
(4) Dow Corning, Düsseldorf

## Patentansprüche

1. Verwendung von Sonnenschutzformulierungen, welche anorganische Lichtschutzfilter enthalten, die im UVA- und UVB-Bereich sowie im IR-und sichtbaren Bereich Strahlung absorbieren, zur Herstellung eines Arzneimittels zur Prophylaxe gegen herpititforme Erkrankungen der Haut.

2. Verwendung von Sonnenschutzformulierungen, welche anorganische Lichtschutzfilter enthalten, gemäß Anspruch 1 zur Herstellung eines Arzneimittels zur Prophylaxe gegen herpititforme Erkrankungen der Haut im Mund- und Lippenbereich.

3. Verwendung von Sonnenschutzformulierungen, welche anorganische Lichtschutzfilter enthalten, gemäß mindestens einem der Ansprüche 1 oder 2 zur Herstellung eines Arzneimittels zur Prophylaxe gegen herpititformen Erkrankungen der Haut hervorgerufen durch Viren der Gruppe Herpes simplex und Herpes labialis.

4. Verwendung von Sonnenschutzformulierungen gemäß mindestens einem der vorhergehenden Ansprüche, **dadurch gekennzeichnet, dass** die Sonnenschutzformulierungen im sichtbaren Wellenlängenbereich reflektierende und/oder absorbierende Pigmente und/oder Farbstoffe, vorzugsweise Interferenzpigmente enthalten.

## Claims

1. Use of sunscreen formulations which comprise inorganic light protection filters which absorb radiation in the UVA and UVB region and also in the IR and visible region for the preparation of a medicament for prophylaxis against herpetic diseases of the skin.

2. Use of sunscreen formulations which comprise inorganic light protection filters according to Claim 1 for the preparation of a medicament for prophylaxis against herpetic diseases of the skin in the mouth and lip area.

3. Use of sunscreen formulations which comprise inorganic light protection filters according to at least one of Claims 1 or 2 for the preparation of a medicament for prophylaxis against herpetic diseases of the skin caused by viruses of the group consisting of Herpes simplex and Herpes labialis.

4. Use of sunscreen formulations according to at least one of the preceding claims, **characterized in that** the sunscreen formulations comprise pigments and/or dyes which reflect and/or absorb in the visible wavelength region, preferably interference pigments.

## Revendications

1. Utilisation de formulations pour la protection solaire contenant des filtres de protection contre la lumière inorganiques, absorbant le rayonnement dans les régions A et B ultraviolettes, ainsi que dans les domaines IR et du visible, pour la fabrication d'un médicament destiné à la prophylaxie des maladies herpétiques de la peau.

2. Utilisation de formulations pour la protection solaire contenant des filtres de protection contre la lumière inorganiques, selon la revendication 1, pour la fabrication d'un médicament destiné à la prophylaxie des maladies herpétiques de la peau dans la région de la bouche et des lèvres.

3. Utilisation de formulations pour la protection solaire contenant des filtres de protection contre la lumière inorganiques, selon au moins l'une des revendications 1 et 2, pour la fabrication d'un médicament destiné à la prophylaxie des maladies herpétiques de la peau provoquées par les virus du groupe Herpes simplex et Herpes labialis.

4. Utilisation de formulations pour la protection solaire selon au moins l'une des revendications précédentes, **caractérisée en ce que** les formulations pour la protection solaire dans les bandes de longueurs d'onde visible contiennent des pigments réfléchissants et/ou des absorbants et/ou des colorants, de préférence des pigments d'interférence.
